(19) Europäisches Patentamt
European Patent Office
Office européen des brevets

(11) Publication number: **0 196 632 B1**

(12) # EUROPEAN PATENT SPECIFICATION

(45) Date of publication of patent specification: **19.12.90**

(51) Int. Cl.$^5$: **A 61 K 31/19**, A 61 K 47/00, A 61 K 9/70

(21) Application number: **86104255.4**

(22) Date of filing: **27.03.86**

(54) **Composition for transdermal drug delivery.**

(30) Priority: **03.04.85 US 719335**

(43) Date of publication of application: **08.10.86 Bulletin 86/41**

(45) Publication of the grant of the patent: **19.12.90 Bulletin 90/51**

(84) Designated Contracting States: **AT BE CH DE FR GB IT LI NL SE**

(56) References cited:
**US-A-3 640 741**
**US-A-4 307 717**

(73) Proprietor: **TSUMURA INTERNATIONAL INC.**
**104 Peavey Road**
**Chaska Minnesota 55318 (US)**

(72) Inventor: **Reever, Richard**
**Route 1 Box 93A**
**Plato Minnesota 55370 (US)**
Inventor: **Lundmark, Larry**
**2925 84th Avenue N.**
**Brooklyn Park Minnesota 55444 (US)**
Inventor: **Kapsner, Timothy**
**5240 Abbott Avenue South**
**Minneapolis Minnesota 55410 (US)**

(74) Representative: **Strehl, Schübel-Hopf, Groening**
**Maximilianstrasse 54 Postfach 22 14 55**
**D-8000 München 22 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 196 632 B1

Courier Press, Leamington Spa, England.

## Description

Background of the invention

1. Field of the invention

This invention relates to a composition for transdermal drug delivery which is particularly useful for topical application to a person's skin of drugs such as salicyclic acid or hydroquinone.

2. Description of the prior art

Sterculia gum, also known as gum karaya, is a hydrophilic colloid made from exudate of the Sterculia Urens tree. It is a complex polysaccharide gum with a molecular weight of almost 10 million. It contains approximately 8% acetyl groups and 37% uronic acid residues, being comprised mainly of D-galacturonic acid, D-galactose and 1-rhamnose.

Sterculia gum has many known applications in the medical field. It has been used as a treatment for chronic skin ulcers, as a denture adhesive and as a bulk laxative. It has also been used as a skin adhesive with principal application as an adhesive ring for attaching stoma bags to patients.

Sterculia gum has been used in combination with hydric alcohols as an electrode for medical monitoring and stimulation through a person's skin. The mixture of the natural organic polysaccharide and hydric alcohol forms an adhesive capable of conducting electricity.

Sterculia gum has been used in combination with cross-linking agents such as propylene glycol plus a non-reactive water soluble carrier such as glycerol. Medication or cosmetic additives are dissolved in the carrier. U.S. Patent No. 3,640,741, teaches that the mixture of gum and cross-linking agent forms a gel which traps the carrier and dissolved additive in the gel. The gel will dissolve slowly in a liquid medium such as mammalian body fluid, thereby providing for slow timed release of medication additives in the body or cosmetic additives on the surface of a person's skin.

Sterculia gum has been used in combination with a synthetic resin such as polyacrylic acid or polyacrylamide plus a carrier such as propylene glycol or glycerol. U.S. Patent No. 4,307,717, teaches that the synthetic resin preserves dimensional stability of the delivery patch, even though the patch is irradiated to prevent unwanted growth of bacteria or fungus in and on the patch. The synthetic resin also preserves the dimensional stability of the delivery patch when the patch is in contact with body fluids appearing on the surface of the skin. The carrier embodied in the patch will deliver solubilized medicaments to the surface of the skin as the patch slowly absorbs body fluids from the skin surface. The US patent No. 4,307,717 teaches that the medicament may be a keratolytic agent such as salicyclic acid, but requires the presence of the synthetic resin to protect the patch during irradiation. The Hymes patent does not teach the use of polyethylene glycol.

Salicylic acid has been used for many years to treat common warts. The most common commercial form is a flexible collodion (e.g. Compound W, available from White Hall Laboratories, Division of American Home Products Corporation, New York City, New York 10017). The salicylic acid is dissolved in a vehicle consisting of nitrocellulose and solvents such as alcohol and ether. The flexible collodion is applied to a wart where it dries to a white crust. The principal disadvantages include the messiness of the application procedure and the precipitation of the salicylic acid upon evaporation of the solvents.

A less common method of applying salicyclic acid to the skin involves suspending salicylic acid at about 40% by weight concentration in an adhesive base which is adhered to a paper or cloth backing. This material is cut to size and held against the skin by waterproof tape.

Salicylic acid may also be used in treating hyperkeratotic conditions such as corns and calluses. The salicylic acid softens and destroys the stratum corneum by increasing endogenous hydration which causes the cornified epithelium to swell, soften, macerate and finally desquamate.

Summary of the invention

In accordance with the invention, it has been found that a composition for transdermal drug delivery, having adhesive properties for adhesion to the skin and being sufficiently pliant to conform to the shape of body contours, can be formed from a gelation product of a mixture comprising an organic polysaccharide gum, the amount being in the range of 15 to 55% of the weight of the mixture polyethylene glycol and meta-, para- or ortho- hydroxybenzoic acid (i.e. m-, p- or o-hydroxybenzoic acid), the amount of o-hydroxybenzoic acid being in the range of 3 to 25% of the weight of the mixture.

Based on the following examples, it is surmised that gels formed from mixtures of organic polysaccharides and drug carriers, in the absence of synthetic resins disclosed in the above mentioned US patent 4,307,717 which alter structural integrity, vary in physical characteristics according to the concentration of the carrier. The physical characteristics vary between mushy gels corresponding to high concentrations of carrier and stiff or brittle gels corresponding to low concentrations of carrier. Toward the mushy end of the continuum, a gel is increasingly deformable and tacky (i.e. adhesive). Both deformability and tackiness are desirable in successfully adhering a gel patch to skin. Toward the stiff end of the continuum, a gel is increasingly resilient. This structural integrity is desirable for the handling of the gel patch during its manufacture. Structural integrity is also desirable because when a gel patch is adhering to the skin it adsorbs moisture, thereby degrading the gel patch in the direction of mushiness.

The present invention thus rests on the finding that polyethylene glycol and m-, p- or o-hydroxybenzoic acid combine with polysaccharide gums to form a gel having both desirable tackiness/deformability and desirable structural integrity. In contrast, polyethylene glycol and polysaccharide gums, without m-, p- or o-hydroxybenzoic acid, mostly fail to form gels or form mushy gels lacking structural integrity even at modest concentrations of polyethylene glycol.

The composition for transdermal drug delivery of the present invention is a gelation reaction product of a mixture comprising an organic polysaccharide gum, in an amount of 15 to 55 weight %, based on the weight of the mixture, polyethylene glycol and m-, p- or o-hydroxybenzoic acid the amount of o-hydroxybenzoic acid being in the range of 3 to 25% by weight, based on the weight of the mixture. This mixture is effective in forming a gel having adhesive properties for adhesion to the skin and being sufficiently pliant to conform to the shape of body contours. The organic polysaccharide gum may preferably be Sterculia gum. The hydroxybenzoic acid may be m-hydroxybenzoic acid, p-hydroxybenzoic acid or preferably o-hydroxybenzoic acid (i.e. salicylic acid).

The foregoing gel may be used to delivery solubilized salicylic acid itself to the skin. It may also be used to deliver to the skin a non-hydroxybenzoic acid drug (i.e. a medicament or cosmetic) including hydroquinone.

Brief description of drawings

Fig. 1 shows the results of 5% and 15% salicylic acid delivery vehicles used in treatment of warts.

Description of the preferred and alternative embodiments

In the preferred embodiment, about 5% to 15% powdered salicylic acid, about 45% to 35% powdered Sterculia gum (salicylic acid and Sterculia gum adding up to about 50%), about 25% polyethylene glycol, about 25% propylene glycol and about 0.2% quaternium-15® (available as Dowicil®-200 from Dow Chemical, Midland, Michigan) are placed into a container and mixed at room temperature until a substantially homogeneous slurry is formed. All concentrations or amounts stated herein by percentage are percentage by weight of the mixture prior to the gelation reaction. The homogeneous slurry in the container is warmed slightly on a hot plate and stirred until the slurry begins to thicken slightly. The heating process assists in dissolving the various components and in initiating the gelation reaction.

The slightly thickened mixture is poured onto polymer-coated release paper. Another piece of release paper is placed on top of the mixture and flattened to desired thickness. The sandwich consisting of release paper and mixture may be heated in a convection or radiation oven at 100°C and allowed to gel for 5 minutes. After cooling, the gelation reaction product may be cut to a preferred size of less than 3 mm thickness and less than 25 mm in diameter for treatment of warts.

According to the invention, the amount of salicylic acid varies within the range of 3 to 25 percent of the weight of the mixture. The amount of Sterculia gum varies within the range of 15 to 55 percent of the weight of the mixture.

The polyethylene glycol may be a polymer of ethylene oxide that conforms generally to the formula

$$H(OCH_2CH_2)_nOH$$

where n has some average value in the range of about 4 to 12. For example, the polyethylene glycol may be PEG-4, PEG-6, PEG-8, PEG-10 or PEG-12 (Cosmetic Toiletry and Fragrance Association designations where PEG-n designates

$$H(OCH_2CH_2)_nOH).$$

The preferred embodiment polyethylene glycols are PEG-4 and PEG-6 which are available commercially as Carbowax® 200 and Carbowax ®300, respectively, from Union Carbide, Chicago, Illinois.

In the preferred embodiment, polyethylene glycol and propylene glycol are used. Alternatively, polyethylene glycol and glycerin or polyethylene glycol by itself or in combination with other hydroxybenzoic solvents or materials may be used.

The preferred preservative is about 0.2% quaternium-15® by weight of the mixture, but may also be paraben® or other preservatives in small amounts generally less than 1% of the weight of the mixture.

Example 1

Salicylic acid was varied according to the following: 0, 1, 2, 3, 5, 15, 25 and 35% by weight of the mixture. Stepculia gum was held constant at 35%, while PEG-6 (Carbowax® 300) and propylene glycol made up the remaining portion of the mixture in a 1:1 weight ratio. Without salicyclic acid (i.e. at 0%), no gel formed upon heating the mixture. At 1% and 2% salicylic acid, a gel formed but the gel was deficient in structural integrity and disassociated. At 3, 5, 15 and 25% salicylic acid, a gel formed having desirable tacky and pliant characteristics as well as structural integrity. At 35% salicylic acid, an extremely tough and stiff gel formed and after 30 minutes at room temperature, the salicylic acid precipitated throughout the gel.

Example 2

Salicylic acid was held constant at 15% and Sterculia gum was held constant at 35% by weight of the mixture. Preservative quaternium-15® was held constant at 0.2%. The remaining portion consisted of PEG-6 (Carbowax® 300) and propylene glycol in 1:1, 9:1 and 2:8 ratios by weight. The example was repeated substituting PEG-4 (Carbowax® 200) for PEG-6.

The 1:1 ratio of PEG-4 or PEG-6 resulted in the most desirable combination of tackiness/pliancy and structural integrity. The 9:1 ratio resulted in a

patch having increased structural integrity but decreased tackiness/pliancy. The 2:8 ratio resulted in a patch having increased tackiness/pliancy but decreased structural integrity. In all three cases, however, the mixture formed a usable transdermal drug delivery vehicle upon gelation.

Example 3

Salicylic acid was held constant at 15% by weight of the mixture. Preservative quaternium-15® was held constant at 0.2%. Sterculia gum was varied according to the following: 10, 15 and 50% while PEG-6 and propylene glycol at 1:1 weight ratio made up the remaining portion of the mixture. Salicylic acid was lowered to 5%, Sterculia gum varied at 55 and 60%, with preservative and PEG-6/propylene glycol ratio as above.

At 10% Sterculia gum, a gel barely formed upon heating, but was deficient in structural integrity and easily disassociated. At 15 and 50% Sterculia gum, a gel formed having desirable tacky/pliant characteristics as well as structural integrity. At 55% Sterculia gum, a gel formed which was tough but deficient in tackiness/pliant characteristics. At 60% Sterculia gum, a gel did not form, with significant amounts of the powdered salicylic acid and powdered Sterculia gum remaining undissolved by the liquid.

Example 4 (Comparative Example)

In the absence of salicylic acid, a gel did not form with more than 25% by weight of mixture of PEG-4, even if propylene glycol or glycerine in 1:1 ratio to PEG-4 were also included.

Example 5

Salicylic acid at 15% and Sterculia gum at 35% by weight of the mixture were held constant. 50% propylene glycol was compared to 25% propylene glycol and 25% PEG-300. At 50% propylene glycol, a gel formed but which was softer and not as strong as the gel resulting from 25% propylene glycol and 25% PEG-6.

Example 6

The mixture consisted of 15% meta-hydroxybenzoic acid, 24.9% PEG-6, 24.9% propylene glycol, 35% Sterculia gum and 0.2% quaternium-15®. Alternatively, para-hydroxybenzoic acid was substituted for meta-hydroxybenzoic acid, the amounts of each component adjusted to yield the same percentages by weight. The resulting gelation reactions for each of the meta- and para-hydroxybenzoic acid mixtures requires slightly additional heating than that for ortho-hydroxybenzoic acid (i.e. salicylic acid). As the hydroxyl group moves further away from the carboxylic acid group, the gel increases in structural integrity but decreases in tackiness/pliancy.

Example 7

Hydroquinone at 1%, PEG-300 at 24.4%, propylene glycol at 24.4%, Sterculia gum at 35%, m-hydroxybenzoic acid at 15% and quaternium-15® at 0.2% by weight of mixture resulted in a gel with good tackiness/pliancy and structural integrity. Thus, the use of m-, p- or o-hydroxybenzoic acid, especially meta- and para- which do not readily react with skin, might be useful as part of a delivery vehicle for non-hydroxybenzoic drugs such as hydroquinone.

Example 8

A clinical evaluation was conducted comparing 5% salicylic acid and 15% salicylic acid to a placebo control according to the following formulations:

| 5%/15% Salicylic Acid | Control |
|---|---|
| 5%/15% Salicylic Acid | 35% Sterculia Gum |
| 45%/3[illegible] | |
| 24.9%/24.9% PEG-6 | 7.5% Water |
| 24.9%/24.9% Propylene Glycol | |
| 0.2%/0.2% Quaternium-15® | |

The placebo control involved a variant formulation in order to produce a gelled patch in the absence of salicylic acid.

The clinical research was conducted at two sites in the United States. The investigators for both studies were practicing professionals active in dermatology and the treatment of warts. 119 people completed the study with a total of 377 warts. The study lasted 12 weeks and included regular visits to the investigator for evaluation. Effectiveness of the drug was measured in terms of significant reduction in the size of the wart or in its complete removal versus no change in size. Total effectiveness of the treatment was compared with the placebo. From prior wart removal clinical studies and discussion in the literature, a treatment is termed effective if the difference between treatment and control cured and improved rate is greater than 20% for the approximate test population size used here.

Below are general effectiveness ratings for common warts (V.V. warts) for both 5 and 15% salicylic acid treatments and placebo at 12 weeks:

| Treatment | % Cured and Improved |
|---|---|
| 5% Salicylic Acid | 72 |
| 15% Salicylic Acid | 85 |
| Placebo Control | 40 |

The differential in cure between treatment and control is significantly greater than 20% at 12 weeks. Fig. 1 shows the results throughout the 12 week period.

From the foregoing, it will be obvious to those skilled in the art that various modifications in the above described methods and materials can be made without departing from the spirit of the invention. Accordingly, the invention may be

embodied in other specific forms without departing from the spirit of the invention. Present embodiments are to be considered in all respects as illustrative and not restrictive, the scope of the invention being indicated by the appended claims rather than by the foregoing descriptions, and changes which come within the meaning and range of equivalency of the claims are therefore intended to be embraced therein.

**Claims for the Contracting States: BE, CH, DE, FR, GB, IT, LI, NL, SE**

1. As a composition for transdermal drug delivery, the gelation reaction product of a mixture comprising

(A) an organic polysaccharide gum, the amount being in the range of 15 to 55% of the weight of the mixture;

(B) polyethylene glycol; and

(C) m-, p- or o-hydroxybenzoic acid, the amount of o-hydroxybenzoic acid being in the range of 3 to 25% of the weight of the mixture.

2. The gelation reaction product of claim 1 wherein the organic polysaccharide gum is Sterculia gum.

3. The gelation reaction product of claim 1 wherein component (C) is o-hydroxybenzoic acid and such o-hydroxybenzoic acid is substantially solubilized in the reaction product for delivery to skin.

4. The gelation reaction product of claim 1 wherein the mixture further comprises a solubilized non-hydroxybenzoic acid drug, preferably hydroquinone.

5. The gelation reaction product of any of the claims 1 to 4 wherein the mixture further comprises propylene glycol and/or glycerin.

6. The gelation reaction product of any of the claims 1 to 5 wherein the polyethylene glycol is a polymer of ethylene oxide that conforms generally to the formula

$$H(OCH_2CH_2)_nOH$$

where n has some average value in the range of about 4 to 12, preferably in the range of about 4 to 6.

7. The gelation reaction product of claim 6 wherein the polyethylene glycol conforms to said general formula wherein n is 4 or 6.

8. The gelation reaction product of any of the claims 1 to 7 wherein the mixture further comprises a preservative in an amount less than 1 percent of the weight of the mixture.

9. The gelation reaction product of any of the claims 1 to 8 wherein the gelation reaction product is less than 25 mm in diameter, and preferably is in the form of a sheet less than 3 mm thick.

**Claims for the Contracting State: AT**

1. Process for preparing a composition for transdermal drug delivery, characterised by mixing and reacting

(A) an organic polysaccharide gum, the amount being in the range of 15 to 55% of the weight of the mixture;

(B) polyethylene glycol; and

(C) m-, p- or o-hydroxybenzoic acid, the amount of o-hydroxybenzoic acid being in the range of 3 to 25% of the weight of the mixture.

2. The process of claim 1 wherein the organic polysaccharide gum is Sterculia gum.

3. The process of claim 1 wherein component (C) is o-hydroxybenzoic acid and such o-hydroxybenzoic acid is substantially solubilized in the reaction product for delivery to skin.

4. The process of claim 1 wherein further a solubilized non-hydroxybenzoic acid drug, preferably hydroquinone, is added.

5. The process of any of the claims 1 to 4 wherein further propylene glycol and/or glycerin is added.

6. The process of any of the claims 1 to 5 wherein the polyethylene glycol is a polymer of ethylene oxide that conforms generally to the formula

$$H(OCH_2CH_2)_nOH$$

where n has some average value in the range of about 4 to 12, preferably in the range of about 4 to 6.

7. The process of claim 6 wherein the polyethylene glycol conforms to said general formula wherein n is 4 or 6.

8. The process of any of the claims 1 to 7 wherein further a preservative is added in an amount less than 1 percent of the weight of the mixture.

9. The process of any of the claims 1 to 8 wherein the gelation reaction product is further shaped to have a diameter of less than 25 mm in diameter, and preferably is shaped in the form of a sheet less than 3 mm thick.

**Patentansprüche für die Vertragsstaaten: BE, CH, DE, FR, GB, IT, LI, NL, SE**

1. Gelbildungs-Reaktionsprodukt einer Mischung, enthaltend

(A) einen organischen Polysaccharidgummi, dessen Gehalt im Bereich von 15 bis 55% des Mischungsgewichts liegt;

(B) Polyethylenglykol und

(C) m-, p- oder o-Hydroxybenzoesäure, wobei der Gehalt an o-Hydroxybenzoesäure im Bereich von 3 bis 25% des Mischungsgewichtes liegt, als Mittel zur transdermalen Arzneimittelabgabe.

2. Gelbildungs-Reaktionsprodukt nach Anspruch 1, worin der organische Polysaccharidgummi Sterculiagummi ist.

3. Gelbildungs-Reaktionsprodukt nach Anspruch 1, worin die Komponente (C) o-Hydroxybenzoesäure ist, und diese o-Hydroxybenzoesäure im wesentlichen in dem Reaktionsprodukt zur Abgabe an die Haut gelöst ist.

4. Gelbildungs-Reaktionsprodukt nach Anspruch 1, worin die Mischung außerdem ein

gelöstes Arzneimittel, das nicht Hydroxybenzoesäure ist, vorzugsweise Hydrochinon, enthält.

5. Gelbildungs-Reaktionsprodukt nach einem der Ansprüche 1 bis 4, worin die Mischung außerdem Propylenglykol und/oder Glyzerin enthält.

6. Gelbildungs-Reaktionsprodukt nach einem der Ansprüche 1 bis 5, worin das Polyethylenglykol ein Polymeres aus Ethylenoxid ist, das allgemein der Formel

$$H(OCH_2CH_2)_nOH$$

entspricht, in der n einen mittleren Wert im Bereich von etwa 4 bis 12, vorzugsweise im Bereich von etwa 4 bis 6, hat.

7. Gelbildungs-Reaktionsprodukt nach Anspruch 6, worin das Polyethylenglykol dieser allgemeinen Formel, in der n 4 oder 6 ist, entspricht.

8. Gelbildungs-Reaktionsprodukt nach einem der Ansprüche 1 bis 7, worin die Mischung außerdem ein Konservierungsmittel in einem Gehalt, der weniger als 1% des Mischungsgewichts ist, enthält.

9. Gelbildungs-Reaktionsprodukt nach einem der Ansprüche 1 bis 8, worin das Gelbildungs-Reaktionsprodukt einen Durchmesser von weniger als 25 mm hat und vorzugsweise die Form einer Folie mit weniger als 3 mm Dicke hat.

**Patentansprüche für den Vertragsstaat: AT**

1. Verfahren zur Herstellung einer Zusammensetzung zur transdermalen Arzneimittelabgabe, gekennzeichnet durch das Vermischen und Umsetzen

(A) eines organischen Polysaccharidgummis, wobei dessen Gehalt im Bereich von 15 bis 55% des Mischungsgewichts liegt;

(B) Polyethylenglykol; und

(C) m-, p- oder o-Hydroxybenzoesäure, wobei der Gehalt an o-Hydroxybenzoesäure im Bereich von 3 bis 25% des Mischungsgewichtes liegt.

2. Verfahren nach Anspruch 1, bei dem der organische Polysaccharidgummi Sterculiagummi ist.

3. Verfahren nach Anspruch 1, bei dem die Komponente (C) o-Hydroxybenzoesäure ist, und diese o-Hydroxybenzoesäure im wesentlichen in dem Reaktionsprodukt zur Abgabe an die Haut gelöst ist.

4. Verfahren nach Anspruch 1, bei dem außerdem ein gelöstes Arzneimittel, das nicht Hydroxybenzoesäure ist, vorzugsweise Hydrochinon, zugegeben wird.

5. Verfahren nach einem der Ansprüche 1 bis 4, bei dem außerdem Propylenglykol und/oder Glyzerin zugegeben werden.

6. Verfahren nach einem der Ansprüche 1 bis 5, bei dem das Polyethylenglykol ein Polymeres aus Ethylenoxid ist, das allgemein der Formel

$$H(OCH_2CH_2)_nOH$$

entspricht, in der n einen mittleren Wert im Bereich von etwa 4 bis 12, vorzugsweise im Bereich von etwa 4 bis 6, hat.

7. Verfahren nach Anspruch 6, bei dem das Polyethylenglykol dieser allgemeinen Formel, in der n 4 oder 6 ist, entspricht.

8. Verfahren nach einem der Ansprüche 1 bis 7, bei dem außerdem ein Konservierungsmittel in einem Gehalt der weniger als 1% des Mischungsgewichts ist, zugegeben wird.

9. Verfahren nach einem der Ansprüche 1 bis 8, bei dem das Gelbildungs-Reaktionsprodukt zudem so geformt wird, daß es einen Durchmesser von weniger als 25 mm hat und vorzugsweise in Gestalt einer Folie mit einer Dicke von weniger als 3 mm geformt wird.

**Revendications pour les Etats Contractants: BE, CH, DE, FR, GB, IT, LI, NL, SE**

1. Comme composition pour l'apport transdermal d'un médicament, le produit de la réaction de gélification d'un mélange comprenant

(A) une gomme polysaccharidique organique, dont la quantité est dans un intervalle de 15 à 55% du poids du mélange;

(B) du polyéthylèneglycol; et

(C) de l'acide m-, p- ou o-hydroxybenzoïque, la quantité d'acide o-hydroxybenzoïque dans un intervalle de 3 à 25% du poids du mélange.

2. Produit de la réaction de gélification de la revendication 1, dans lequel la gomme polysaccharidique organique est la gomme de Sterculia.

3. Produit de la réaction de gélification de la revendication 1, dans lequel le composant (C) est l'acide o-hydroxybenzoïque et cet acide o-hydroxybenzoïque est essentiellement solubilisé dans le produit réactionnel pour apport à la peau.

4. Produit de la réaction de gélification de la revendication 1 dans lequel le mélange comprend en outre un médicament solubilisé non apparenté à l'acide hydroxybenzoïque, de préférence l'hydroquinone.

5. Produit de la reaction de gélification de l'une quelconque des revendications 1 à 4 dans lequel le mélange comprend en outre du propylèneglycol et/ou de la glycérine.

6. Produit de la réaction de gélification de l'une quelconque des revendications 1 à 5 dans lequel le polyéthylèneglycol est un polymère d'oxyde d'éthylène qui se conforme généralement à la formule

$$H(OCH_2CH_2)_nOH$$

où n présente une valeur moyenne dans un intervalle d'environ 4 à 12, de préférence dans un intervalle d'environ 4 à 6.

7. Produit de la réaction de gélification de la revendication 6, dans lequel le polyéthylèneglycol se conforme à ladite formule générale, où n vaut 4 ou 6.

8. Produit de la réaction de gélification de l'une quelconque des revendications 1 à 7 dans lequel le mélange comprend en outre un agent de conservation en une quantité inférieure à 1% en poids du mélange.

9. Produit de la réaction de gélification de l'une quelconque des revendications 1 à 8, dans lequel le produit de la réaction de gélification a un diamètre inférieur 25 mm, et de préférence est sous la forme d'une feuille d'une épaisseur inférieure à 3 mm.

**Revendications pour l'Etat Contractant: AT**

1. Procédé de préparation d'une composition pour l'apport transdermal d'un médicament, caractérisé en ce qu'on mélange et en ce qu'on fait réagir

(A) une gomme polysaccharidique organique, dont la quantité est dans un intervalle de 15 à 55% en poids du mélange;

(B) du polyéthylèneglycol; et

(C) de l'acide m-, p- ou o-hydroxybenzoïque, la quantité d'acide o-hydroxybenzoïque étant dans un intervalle de 3 à 25% en poids du mélange.

2. Procédé de la revendication 1, dans lequel la gomme polysaccharidique organique est la gomme de Sterculia.

3. Procédé de la revendication 1, dans lequel le composant (C) est l'acide o-hydroxybenzoïque et cet acide o-hydroxybenzoïque est essentiellement solubilisé dans le produit réactionnel aux fins d'apport à la peau.

4. Procédé de la revendication 1, dans lequel on ajoute encore un médicament solubilisé nonapparenté à l'acide hydroxybenzoïque, de préférence l'hydroquinone.

5. Procédé de l'une quelconque des revendications 1 à 4, dans lequel on ajoute en outre du propylèneglycol et/ou de la glycérine.

6. Procédé de l'une quelconque des revendications 1 à 5, dans lequel le polyéthylèneglycol est un polymère d'oxyde d'éthylène qui se conforme généralement à la formule

$$H(OCH_2CH_2)_nOH$$

où n présente une valeur moyenne située dans un intervalle allant d'environ 4 à 12, de préférence dans l'intervalle d'environ 4 à 6.

7. Procédé de la revendication 6, dans lequel le polyéthylèneglycol se conforme à ladite formule générale où n vaut 4 ou 6.

8. Procédé de l'une quelconque des revendications 1 à 7, dans lequel on ajoute encore un agent de conservation en une quantité inférieure à 1% en poids du mélange.

9. Procédé de l'une quelconque des revendications 1 à 8, dans lequel le produit de la réaction de gélification est en outre façonné pour lui donner un diamètre inférieur à 25 mm et de préférence façonné en feuille ayant une épaisseur inférieure à 3 mm.

*Fig. 1*

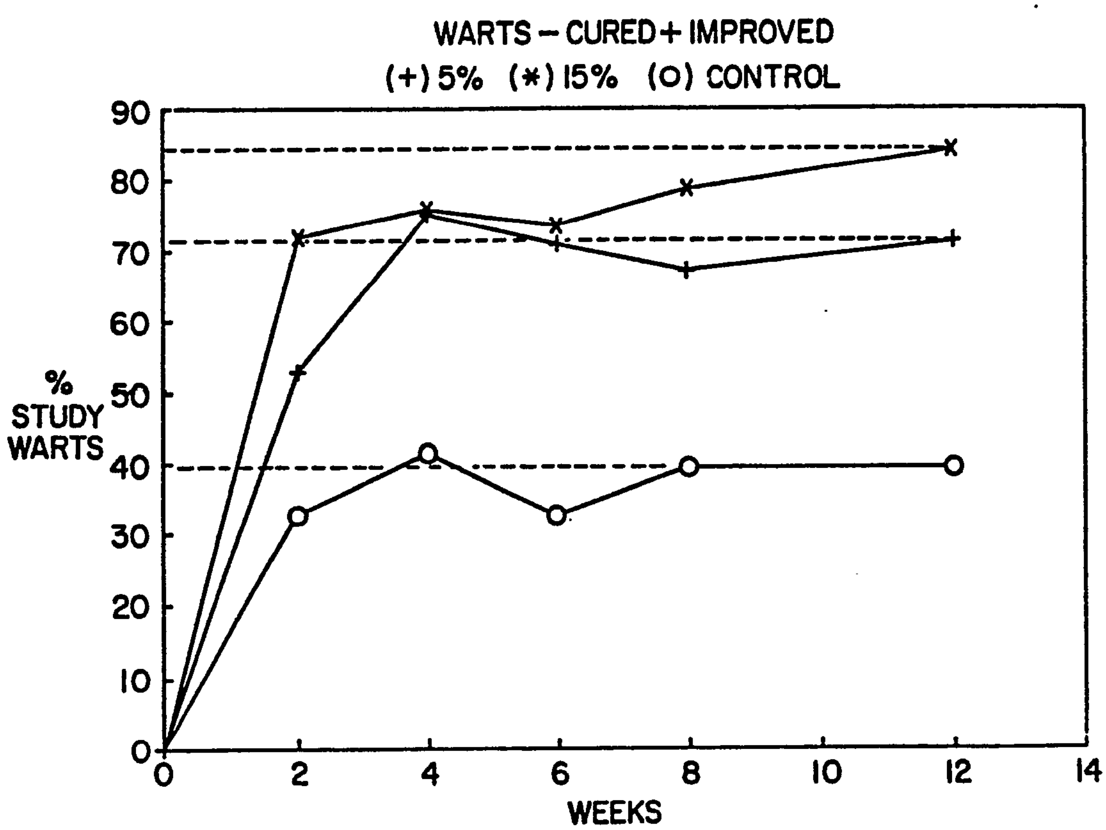
WARTS – CURED + IMPROVED
(+) 5% (*) 15% (O) CONTROL
90
80
70
60
50
40
30
20
10
0
% STUDY WARTS
0
2
4
6
8
10
12
14
WEEKS